# EUROPEAN PATENT SPECIFICATION

(11) **EP 4 428 871 B1**
(45) Date of publication and mention of the grant of the patent: **01.10.2025**
(21) Application number: 23461531.8
(22) Date of filing: 09.03.2023
(51) Int. Cl.: G16H 30/40, G16H 50/20, G16H 50/50, A61B 5/02, A61B 6/50

(54) **A METHOD OF TRAINING AN ARTIFICIAL DEEP NEURAL NETWORK FOR ESTIMATION OF HEMODYNAMIC PARAMETER, A METHOD OF ESTIMATION OF HEMODYNAMIC PARAMETER, COMPUTER PROGRAM PRODUCTS AND COMPUTER SYSTEMS**
VERFAHREN ZUM TRAINIEREN EINES KÜNSTLICHEN NEURONALEN NETZWERKS ZUR SCHÄTZUNG EINES HÄMODYNAMISCHEN PARAMETERS, VERFAHREN ZUR SCHÄTZUNG EINES HÄMODYNAMISCHEN PARAMETERS, COMPUTERPROGRAMMPRODUKTE UND COMPUTERSYSTEME
PROCÉDÉ D'APPRENTISSAGE D'UN RÉSEAU NEURONAL PROFOND ARTIFICIEL POUR L'ESTIMATION D'UN PARAMÈTRE HÉMODYNAMIQUE, PROCÉDÉ D'ESTIMATION D'UN PARAMÈTRE HÉMODYNAMIQUE, PRODUITS DE PROGRAMME INFORMATIQUE ET SYSTÈMES INFORMATIQUES

(43) Date of publication of application: 11.09.2024
(73) Proprietor: HEMOLENS DIAGNOSTICS SPÓLKA Z OGRANICZONA ODPOWIEDZIALNOSCIA, 54-202 Wroclaw (PL)
(72) Inventor: ZIEBA, MACIEJ, Wroclaw (PL); KONOPCZYNSKI, Tomasz, Wroclaw (PL); RYGIEL, Patryk, Wroclaw (PL); PLUSZKA, Pawel, Wroclaw (PL)
(74) Representative: Bury & Bury

(56) References cited:
- WO-A2-2016/075331
- LI: "Prediction of 3D Cardiovascular hemodynamics before and after coronary artery bypass surgery via deep learning", COMMUN BIOL, vol. 4, 22 January 2021 (2021-01-22), Li,, pages 1 - 12, XP093053274
- JAVADOV AYDIN: "PointNet ++: Deep Hierarchical Feature Learning on Point Sets in a Metric Space [Paper Go-Through] | by Aydin Javadov | Medium", 30 August 2022 (2022-08-30), pages 1 - 15, XP093053275, Retrieved from the Internet <URL:https://medium.com/@ajavadov99/pointnet-deep-hierarchical-feature-learning-on-point-sets-in-a-metric-space-paper-go-through-b9121e559549> [retrieved on 20230610]
- HE TONG ET AL: "GeoNet: Deep Geodesic Networks for Point Cloud Analysis", 2019 IEEE/CVF CONFERENCE ON COMPUTER VISION AND PATTERN RECOGNITION (CVPR), IEEE, 15 June 2019 (2019-06-15), pages 6881 - 6890, XP033687200, DOI: 10.1109/CVPR.2019.00705

## Description

### FIELD OF THE INVENTION

The invention concerns a method of training an artificial deep neural network to estimate a hemodynamic parameter, a method of estimation of hemodynamic parameter with artificial deep neural network, computer program products implementing methods according to the invention and computer systems adapted to implement a method according to the invention.

### BACKGROUND ART

Cardiovascular disease is one of the main causes of death in the world. With developments in Computer Tomography (CT), non-invasive approaches for accurate diagnosis in patients with suspected ischemic heart disease, became possible.

CT scans are commonly represented as a 3D volume image. Such a 3D volume image represents a physical quantity as a function of three spatial coordinates. In a digital volume image, each sample (voxel) represents this quantity measured at a specific location. The image is made by a spatial sequence of 2D slices that include the object of interest. Typically, a slice is represented as an image matrix of pixels (X and Y coordinates). The slice number indicates the Z-coordinate.

Interpretation of CT images and diagnosis of cardiovascular disease is a non-trivial task, which requires highly trained physicians. A common problem is that the interpretation of projections visible on CT images requires a highly skilled radiologist and diagnosing of a cardiovascular disease takes a skilled cardiologist. People, who have competences in both fields are in short supply. Therefore, computer-aided techniques are needed.

One way to diagnose cardiac pathologies, with the aid of a computer, is to produce a diagnosis-assisting 3D model of blood vessels. Especially, in the field of cardiac pathologies, a detailed 3D model of coronary arteries is required. A detailed model allows precise estimation of patient-specific anatomical and functional features, such as hemodynamic parameters. In the state of the art, there are numerous techniques utilizing computational fluid dynamics (CFD) for blood flow simulation. The geometry of a vessel tree, presented in the 3D volumetric image, can be represented as a subset of voxels of the 3D image, a surface mesh, a volumetric mesh, or as a point cloud. An example of a CFD technique used for calculation of the fractional flow reserve (FFR) out of a 3D model of coronary arteries from Coronary Computed Tomography Angiography (CCTA) is disclosed in the European patent EP3820357. Generally, CFD simulations configured to return FFR or other hemodynamic parameter or parameters of a patient under test. Another example is given in an article "Prediction of 3D Cardiovascular hemodynamics before and after coronary artery bypass surgery via deep learning" by Gaoyang Li e. al., doi.org/10.1038/542003-020-01638-1.

CFD simulations are an excellent alternative for manual diagnostics done by physician analyzing CT images or even for invasive measurements. CFD simulations are run using geometric models of blood vessel or blood vessel tree. The geometric models need to be extracted from volume image of patients, usually DICOM images (Digital Imaging and Communications in Medicine is the standard for the communication and management of medical imaging information and related data). There are known methods of automatic or semi-automatic extraction of geometric models from a 3D volumetric image of a patient, as well as methods of enhancing them.

Publication CN114399608 of patent application discloses a time-varying flow field super-resolution reconstruction method for hemodynamic simulation. The method comprises the following steps: step (1) data set production: performing blood vessel simulation by using SimVascular software, and constructing a time-varying flow field data set through the steps of image acquisition, geometric modeling, grid generation and simulation; (2) velocity field feature extraction: extracting data set input data features through PointNet, and generating a 1024-dimensional feature vector fv; (3) time and resistance value feature extraction: extracting time and resistance value feature vectors of input data through a resistance, value-time encoder, and generating a 1024-dimensional feature vector frt; (4) feature decoding is carried out, and a high-time-resolution velocity field is reconstructed; and (5) evaluating and analyzing a reconstruction result, training the network by using amplitude and direction loss functions of the velocity field, and evaluating and analyzing the reconstruction result through an average modulus length error and a relative error. Method of CN114399608 is demonstrated, with respect to modelling a geometry of a segment of artery, which is a relatively simple segment of a vessel tree suitable for modeling with a point cloud. It is a truth, quite universally acknowledged, that point clouds are difficult to process, when they represent complex, elongated structures with multiple branches. It is especially problematic, when at least two separate branches are spatially close to each other. The problem has been discussed by
- Patryk Rygiel, Maciej Zieba, Tomasz Konopczynski in Eigenvector Grouping for Point Cloud Vessel Labeling, Proceedings of the First International Workshop on Geometric Deep Learning in Medical Image Analysis, PMLR 194:72-84, 202,
- He, J. et al. (2020) Learning hybrid representations for automatic 3D vessel centerline extraction, arXiv.org. Available at: https://arxiv.org/abs/2012.07262 (Accessed: February 15, 2023).

A drawback of CFD simulation consists in that it requires considerable time and computational power to be run. Therefore, there is a need for an alternative. A promising alternative is a computer analysis of the geometry of the vessel tree using artificial intelligence trained on a real or artificial data or hybrid thereof. Real data comprise an actual geometry of a vessel tree obtained from the patient and measured hemodynamic parameters. Artificial data include computer generated models of the geometry of the vessel tree and simulated hemodynamic parameters. Hybrid data may comprise real geometries of a vessel tree and simulated hemodynamic parameters. Another kind of hybrid data comprises real and artificial examples.

Systems and methods for artificial intelligence assisted determining individual-specific blood flow characteristics i.e. hemodynamic parameters are disclosed in EP3218872, US2014073976 and US20160166209. Methods include, first, training of an artificial deep neural network, including steps of acquiring individual-specific, real or artificial, geometric model and blood flow characteristics of at least part of the individual's vascular system, creating a feature vector corresponding to the geometric model, according to predefined format, training an artificial deep neural network using said geometric model, said feature vector and said blood flow characteristics. After training is completed, hemodynamic parameters can be estimated by extracting a feature vector from the geometric model and using a trained artificial deep neural network fed with the feature vector to obtain estimation of hemodynamic parameter. Generally, artificial intelligence is used to map certain predefined features extracted from the geometric model to a hemodynamic parameter. The features are related to geometry and include, but are not limited to dimensions of the blood vessels, changes in diameter, size of the lumen length of narrowed parts etc. These features are a design choice of the method, and their specific definition is rarely shared with the public.

### PROBLEM TO BE SOLVED

It is noted that it is difficult and rather not effective to attempt training an artificial deep neural network using whole geometry of the blood vessel tree only in its global context. On the other hand, using only a local context is not effective either. Also, using a predefined vector of physical features to be extracted from the geometry of blood vessel tree is difficult, as said vector cannot be defined in universal manner, adequate for all shapes, in all patients. Additionally, the process of extraction of features is prone to numerical errors and the features need to be defined a priori. Moreover, sets of features known in the art are usually assigned to certain candidate points along the vessel tree and the process of selecting candidate points is also prone to errors.

### SUMMARY OF THE INVENTION

A computer implemented method of training of an artificial deep neural network for estimation of a hemodynamic parameter from a geometry of a blood vessel tree, comprising, according to the invention, includes a step of obtaining a set of geometries of vessel trees, a step of obtaining the hemodynamic parameter corresponding to the geometries within the set, a step of preparing the training data for the artificial deep neural network, a step of training of the artificial deep neural network. The artificial deep neural network according to the invention is an artificial deep neural network (ADNN) having an architecture adapted for point cloud processing with distance based point grouping. The distance is defined as geodesic distance along the blood vessel tree. The step of preparing the training data for the artificial deep neural network comprises representing of the geometry as a point cloud. The parameter or parameters used in the training set further use of the artificial deep neural network for estimation of this hemodynamic parameter from other geometries. Use of a geodesic distance for selecting and grouping neighbors within a point cloud representation allows elastic configuration that adapts itself to actual dynamics of fluid in the vessel tree, without a need for using predefined feature set. This improves accuracy and eliminates errors related to feature selection. Geodesic distance grouping allows analysis of the blood vessel tree in both global and local context and consequently a vector of specific features can be constructed implicitly by the artificial deep neural network itself.

Advantageously, the computer implemented method comprises a step of obtaining a blood vessel tree centerline graph, wherein geodesic distance along the blood vessel tree is measured along the centerline graph. Centerline graph is very adequate for computation of geodesic distance, as it reflects topology of the vessel tree.

Advantageously, obtaining values of hemodynamic parameters comprises using computational fluid dynamics simulation. This approach allows generation of a large training data set without elaborate measurements. This allows more efficient training. The training can be sped up even more, if artificial geometries are used. Hybrid approach, in which real models and artificial models are used can remedy this. In similar manner CFD simulation can be verified with addition of real measurement data.

A computer implemented method of estimation of hemodynamic parameters from a geometry of a blood vessel tree according to the invention involves using an artificial deep neural network, comprising a step of obtaining a geometry of a blood vessel tree and a step of applying an artificial deep neural network for estimation of hemodynamic parameters. The artificial deep neural network, according to the invention, is an artificial deep neural network (ADNN), having an architecture adapted for point cloud processing, with distance based point grouping. The distance is defined as geodesic distance along the blood vessel tree and the geometry is represented as a point cloud. Use of a point cloud enables more elastic modelling of a geometry within a structure of artificial deep neural network. Use of a geodesic distance for selecting and grouping neighbors within a point cloud representation allows elastic configuration that adapts itself to actual dynamics of fluid in the vessel tree without a need for using predefined feature set. This improves accuracy and eliminates errors related to feature selection. Geodesic distance grouping allows analysis of the blood vessel tree in both global and local context and consequently a vector of specific features can be constructed implicitly by the artificial deep neural network itself.

Advantageously, the geodesic distance along the blood vessel tree is measured along the centerline graph.

Advantageously, the step of obtaining geometry comprises loading mesh geometry, a step of transforming the geometry to point cloud and a step of obtaining centerlines.

Advantageously, the processing with artificial deep neural network comprises encoding with at least one centerline set-abstraction block, decoding with at least one decoder block, processing with shared multilayer perceptron block and postprocessing with one-dimensional, convolutional layer.

A computer program product, according to the invention, comprises a set of instructions that when run on a computing system cause it to realize the method of estimation of hemodynamic parameters, according to the invention.

A computer program product, comprising a set of instructions that when run on a computing system cause it to realize the method of training of an artificial deep neural network, according to the invention.

A computing system for extraction of at least one of estimation of hemodynamic parameters from a geometry of a blood vessel tree adapted to realize a method of estimation of hemodynamic parameters, according to the invention.

Advantageously, the system is further adapted to realize training method, according to the invention.

### BRIEF DESCRIPTION OF DRAWINGS

The invention has been described below in detail, with reference to the following figures.
Fig. 1 presents a flow chart of an embodiment of a method of computing hemodynamic parameters in which invention is applicable.
Fig. 2 presents the architecture of the artificial deep neural network model for the hemodynamic parameters' estimation.
Fig. 3 presents a diagram of the centerline set-abstraction (CSA) block which is used in the ADNN model applied in the embodiment of a method, according to the invention.
Fig. 4 presents a diagram of the decoder block which is used in the ADNN model applied in the embodiment of a method according to the invention.
Fig. 5 presents a diagram of the shared multilayer perceptron (MLP) block which is used in the ADNN model applied in the embodiment of a method, according to the invention.
Fig. 6 presents a diagram of the PointNet block 305 which is used, according to the invention.
Fig. 7 presents a specific architecture that was used for the experiments, according to the invention.
Fig. 8 presents a flow chart of an embodiment of the training method, according to the invention.
Fig. 9 presents an example of the synthetic input data.

### DETAILED DESCRIPTION OF EMBODIMENTS OF THE INVENTION

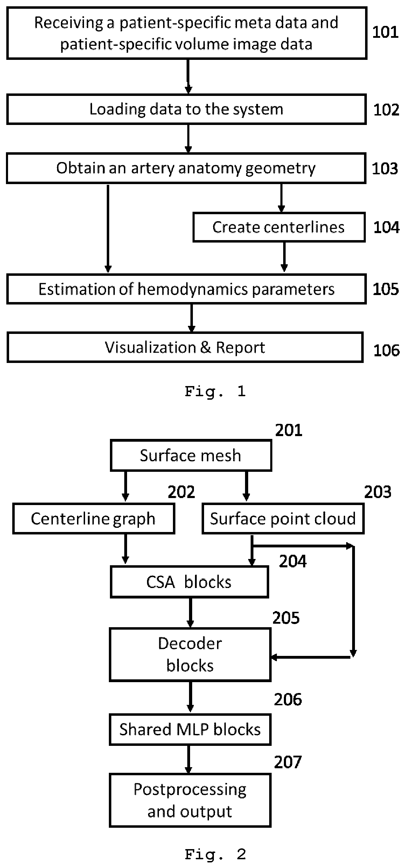

An embodiment of the method of estimation of hemodynamic parameters from a geometry of a blood vessel tree, using an artificial deep neural network, according to the invention, is discussed below in reference to **Fig. 1****,** showing a general flow chart.

Firstly, in the step **101,** patient-specific metadata and patient-specific volume image data are received. Patient-specific metadata is optional, but can improve accuracy. The patient-specific metadata, that can include, but is not limited to features like age, sex, non-invasive blood pressure monitoring, medical history. Patient-specific volume image data is not optional and can be, but is not limited to: a computed tomography (CT) scan, computed tomography angiography (CTA) scan, coronary computed tomography angiography (CCTA) scan, magnetic resonance imaging (MRI) scan. Step **101** of retrieval of the data may involve actual measurements or just downloading the data from external source. It can be retrieved for example from an external drive, Picture archiving and communication system (PACS) or any other means.

Retrieved data are then loaded **102** to computing system executing the method. This system can be a general purpose computer, dedicated, digital processing machine, distributed architecture, virtual machine or cloud resource. It needs to be selected to complete the task in the desired time. In the present embodiment Azure cloud environment is used.

Within the computing system, an artery anatomy geometry is obtained in step **103.** The step **103** of obtaining a geometry just involves loading a surface mesh. However, the step of obtaining **103** can involve geometry format transformation or even obtaining it via volume image segmentation.

The step **103** of obtaining of the geometry can be done manually by a human expert, or automatically, by a computer algorithm, or semi-automatically with a computer algorithm and a human expert. The input artery anatomy geometry can be described as a surface mesh, point cloud surface, or any other relevant data structure. If the data structure is different than a point cloud surface, the artery anatomy geometry data structure must be transformed into a point cloud surface. The points in the point cloud have at least three parameters, which are coordinates in the three dimensional space, and additional number of features. Example features include, but are not limited to, distance to the centerline or geodesic distance from the vessel inlet (beginning). The surface mesh representation makes it easier to determine a centerline graph.

In the next step **104** a centerline graph is then extracted from the artery anatomy geometry. The centerline graph can be extracted manually by a human expert, automatically by a computer algorithm, or semi-automatically with a computer algorithm and a human expert. The centerline graph is described as a connected polygonal chain of 3D points in space.

The centerline graph and artery anatomy geometry are used for an artificial deep learning model described in the invention and discussed later with reference to **Fig. 2-7** estimates the hemodynamic parameters of choice in a step of **105,** especially FFR or pressure drop. Specifically step **105** is realized in steps **204, 205, 206** and **207.**

The hemodynamic parameters are calculated for each point of the artery anatomy geometry point in the point cloud and can be cast on the centerline graph and estimated for each point in the centerline. The hemodynamic parameters can be, but are not limited to pressure drops, a fractional flow reserve (FFR), or any other related hemodynamic parameter. The FFR is defined as a ratio between the pressure in the beginning of the artery and pressure in the measurement point.

The result is then visualized, and a report is produced and send back or presented to the user in the step of outputting **106.**

A general artificial deep neural network (ADNN) model architecture used in the present embodiment of the invention is shown in **Fig. 2****.** The ADNN model shown in Fig. 2 is an example of ADNN model with architecture adapted for point cloud processing. The ADNN model comprises CSA blocks **204,** Decoder blocks **205** and Shared MLP blocks **206.** CSA blocks are fed with centerline graph **202** obtained in the step **104** and geometry of a blood vessel tree represented as a point cloud **203.** In the present embodiment both centerline graph **202** and surface point cloud **203** are obtained from geometry represented initially as a surface mesh **201.**

CSA blocks **204** form an encoder module. The input to the CSA blocks is the centerline graph **202** and the surface point cloud **203.** CSA blocks return a vector which represents the whole surface point cloud **203.**

Decoder blocks **205** are fed with the vector returned by the CSA blocks **204** and with the surface point cloud **203** to return a decoded features point cloud.

The decoded features point cloud is fed to the shared MLP blocks **206** for processing into selected hemodynamic parameter e.g. with 1D convolution layer. Finally, the result is postprocessed and outputted in a block **207.**

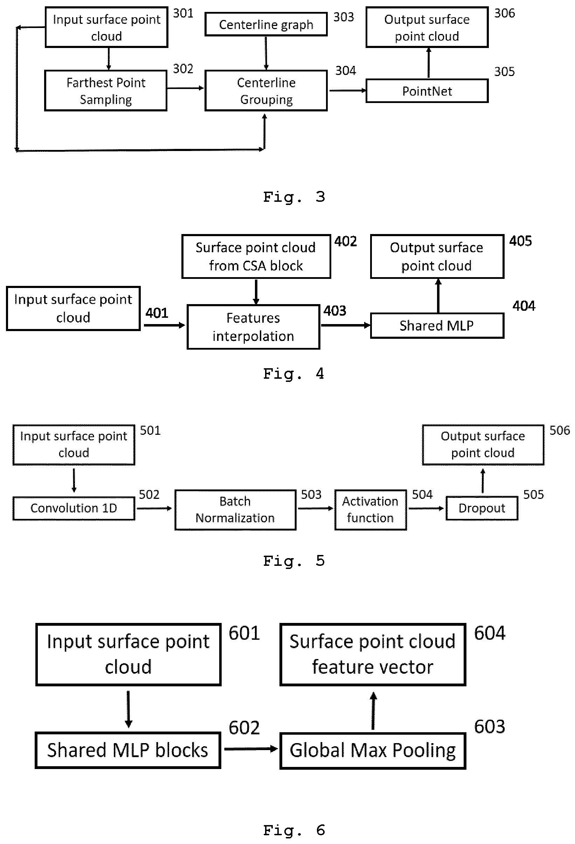

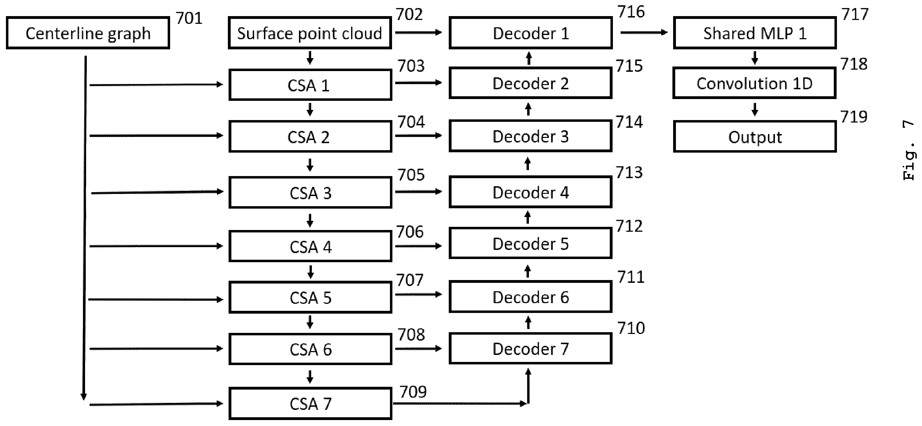

Components of the general ADNN model architecture used in the present embodiment of the invention and shown in **Fig. 2****,** are shown in **Figs. 3-6****.** Corresponding centerline-set-abstraction (CSA) block is presented in **Fig. 3****,** its corresponding decoder block is presented in **Fig. 4****,** its corresponding shared multilayer perceptron (MLP) block is presented in **Fig. 5****.** PointNet block **305** shown in Fig. 3 is described in detail with reference to **Fig. 6****.** The specific configuration of the architecture used for the one other specific embodiment and experiments is shown in **Fig. 7****.**

Specific embodiment is discussed below. In this embodiment the ADNN model takes a centerline graph with *V* number of vertexes, and *E* number of edges **202** and surface point cloud with *Nᵢₙ* number of points and **203** with *Fᵢₙ* per point input features. In one embodiment the centerline graph **701** has an arbitral number of vertexes *V*, and a corresponding number of edges *E.* The number of points *Nᵢₙ* in the surface point cloud **702** is also arbitral and depends on the required resolution. The surface point cloud **702** has *Fᵢₙ* set to five. The first three describe the 3D spatial position, the fourth is the distance to the centerline, and fifth is a geodesic distance from the point to the inlet, where inlet is the beginning of the centerline. Both centerline graph and surface point cloud are extracted from the input vessel geometry represented as a surface mesh **201.**

The CSA block is shown in **Fig. 3****.** The input to the CSA block is an input surface point cloud **301** and centerline graph **303.** The CSA block is parametrized with number of representatives to be sampled *Nₒᵤₜ*, grouping scales *D₁, D₂, ..., Dₙ,* number of points to be grouped for each scale *K₁, K₂, ..., Kₙ* and PointNet **305** configurations. Number n can be selected empirically.

The input surface point cloud **301** is first downsampled to *Nₒᵤₜ* points with the Farthest Point Sampling (FPS) **302** algorithm. The FPS algorithm starts with the representative set R consisting of single point P_0 chosen arbitrarily from the point cloud. The point that is the farthest, in Euclidean distance sense, from the P_0 is extracted and added to the set R. The next point is chosen as the farthest, in Euclidean distance sense, from all the points in the R. This procedure is repeated iteratively until the set R is of required size. Resulting point cloud is to be referred as representative point cloud. In the centerline grouping **304,** the multi-scale grouping is performed independently for each point from the representative point cloud. The multi-scale grouping is defined as performing multiple independent groupings with different parametrizations. Wherein one grouping is a procedure of finding *Kᵢ* neighbors in the surface point cloud for the point from the representative point cloud, according to specified strategy. Wherein strategy is a process of determining an order in some manner of the surface point cloud points, according to the representative point, for which the grouping is performed.

One of the possible grouping strategies is the centerline grouping discussed with reference to the present embodiment. A person skilled in the art, however, is able to suggest alternative mechanisms for local grouping. The advantage of the centerline based grouping is that centerlines are very useful also in generation diagnostic images and therefore are often, already available in the patient specific geometry. Another advantage of centerlines is that it reflects information on topology of the vessel. In this strategy, all surface point cloud points and representative point cloud points get a closest, in the Euclidean distance sense, centerline vertex assigned. For each point in the representative point cloud, geodesic distances between assigned centerline vertex to the representative point and all centerline graph vertexes are computed.

The scale parameter *Dᵢ* is used to extract only centerline vertexes, for which the geodesic distance is smaller than *Dᵢ.* Centerline vertexes extracted in this manner are used to query all surface point cloud points which got these vertexes assigned. Surface point cloud points extracted in this manner are considered a set of representative point's neighbors. The set of neighbors is further downsampled or upsampled to the specified number of neighbors *Kᵢ*. Grouping procedure is conducted in this manner for each scale specified in the CSA parametrization.

Extracted, multi-scale sets of neighbors are processed with PointNet **305** to obtain global neighborhood feature vectors. For each scale, an independent PointNet block is used to extract a feature vector of size *Fᵢ*. The PointNet configurations are passed as a parametrization to the CSA block. The outputs of each scale PointNet are concatenated together point-wise. The output surface point cloud **306** is of size *Nₒᵤₜ* x (*F₁ + F₂ + ... Fₙ*). Wherein *F₁, F₂, ..., Fₙ* are feature vector sizes of each grouping scale. Applicable PointNet processing method is known from publication PointNet++: Deep Hierarchical Feature Learning on Point Sets in a Metric Space by Charles R. Qi, Li Yi, Hao Su, Leonidas J. Guibas (https://doi.org/10.48550/arXiv.1706.02413) and also available in software architecture (https://github.com/charlesq34/pointnet2). This, however, requires modification of distance definition for geodesic distance to use it in the embodiment of the invention. Notably, many other known PointNet based architectures, are not suitable for implementing of distance point grouping and therefore are not suitable for use in the present invention, and implementing geodesic distance point grouping based on centerline or other line reflecting vessel tree topology.

The last CSA block groups all remaining points and creates one global embedding vector which represents the whole surface point cloud **203.** This global embedding vector, together with embedding vectors from corresponding CSA blocks, and the surface point cloud **203** are used as an input to the Decoder blocks **205.**

Details of the PointNet block **305** are presented in **Fig. 6****.** The input to the PointNet block is an input surface point cloud **601** of spatial size *N* x *F.* The input surface point cloud is processed with multiple Shared MLP blocks **602** of specified parametrization. The number of Shared MLP blocks is a design choice. The last Shared MLP block outputs a surface point cloud of spatial size *N* x *Fₒᵤₜ* which is processed with Global Max Pooling **603** to yield single feature vector **604** representing surface point cloud of size *Fₒᵤₜ.*

The number of used CSA blocks, their number of scales, corresponding distance parameters and spatial dimension of the output are a design choice. Stacking more blocks gives more generalizing capabilities, at the cost of more weights and risk of overfitting of the entire model. Stacking blocks allows extraction of local features, capturing fine geometric structures from small neighborhoods at first blocks. Such local features are further grouped into larger units in the next blocks and processed to produce higher level features. It is common to gradually extend the number of output feature channels from previous blocks, usually using the next powers of two, or by multiplying the number of channels by two. The number of blocks with their corresponding number of scale and distance parameters should be selected such that the model takes the entire point cloud into consideration at the last CSA block. Only one block, at least in some vessel tree, may not be able to gather higher level features, and too many blocks may lead to overfitting. In the embodiment described below, these settings were found empirically - as shown in the **Fig 7****.** In the specific embodiment discussed below this number is equal to two: n=2, for all CSA blocks, except for the last one. Accordingly, the blocks are parametrized with *D₁, D₂, K₁, K₂.*

Where the first CSA block **703** takes as an input the centerline graph **701** and a surface point cloud **702.** The parameters D1, D2 of block **703** are set to 0.001 m, 0.002 m, respectively. Numbers K₁ and K₂ are set to K₁=128, K₂=256. The spatial dimension of the output point cloud for block **703** is set to 2048 x (16 + 16).

The next CSA block **704** takes as an input the output of the previous block **703** and the centerline graph **701.** The parameters D1, D2 of block **704** are set to 0.002 m, 0.004 m, respectively. Numbers K₁ and K₂ are set to K₁=16, K₂=32. The spatial dimension of the output point cloud for block **704** is set to 1024 x (32 + 32).

The next CSA block **705** takes as an input the output of the previous block **704** and the centerline graph **701.** The parameters D1, D2 of block **705** are set to 0.004 m, 0.008 m, respectively. Numbers K₁ and K₂ are set to K₁=16, K₂=32. The spatial dimension of the output point cloud for block **705** is set to 512 x (64, 64).

The next CSA block **706** takes as an input the output of the previous block **705** and the centerline graph **701.** The parameters D1, D2 of block **706** are set to 0.008 m, 0.012 m, respectively. Numbers K₁ and K₂ are set to K₁=16, K₂=32. The spatial dimension of the output point cloud for block **706** is set to 256 x (128 + 128).

The next CSA block **707** takes as an input the output of the previous block **706** and the centerline graph **701.** The parameters D1, D2 of block 707 are set to 0.012 m, 0.016 m, respectively. Numbers K₁ and K₂ are set to K₁=16, K₂=32. The spatial dimension of the output point cloud for block **707** is set to 128 x (128 + 128).

The next CSA block **708** takes as an input the output of the previous block **707** and the centerline graph **701.** The parameters D1, D2 of block **708** are set to 0.016 m, 0.032 m, respectively. Numbers K₁ and K₂ are set to K₁=16, K₂=32. The spatial dimension of the output point cloud for block **708** is set to 64 x (128 + 128).

The last CSA block **709** takes as an input the output of the previous block **708** and the centerline graph **701.** The parameter D1 **708** is set to NONE, which is defined as grouping all remaining points. For last CSA block n = 1. The last CSA block aggregates all remaining points and the number K₁ is set to K₁ = 64. The spatial dimension of the output point cloud for block **709** is set to 256.

The decoder module is built out of decoder blocks **205.** The inputs to the decoder blocks are outputs of the CSA blocks **204** and for the last decoder block the surface point cloud **203** as well.

The decoder block is shown in **Fig. 4****.** The input to the decoder block is an input surface point cloud from the previous decoder block **401** and surface point cloud from the respective CSA block **402.** The decoder block is parametrized with Shared MLP configuration.

The input surface point cloud from the previous decoder block **401** and surface point cloud from the respective CSA block **402** are an input to the features interpolation block **403.** In the features interpolation block **403,** per-point features from the input surface point cloud from the previous decoder block are interpolated based on three nearest neighbors, in Euclidean distance sense, onto the surface point cloud from the respective CSA block **402.** Wherein the surface point cloud from the respective CSA block with interpolated features is further processed.

The interpolated surface point cloud is processed with Shared MLP **404** to obtain decoded features point cloud **405.** The MLP block is one of typical mechanisms used for extraction features from bigger set of features - especially for data that is not linearly separable. The Shared MLP configuration is passed as a parametrization to the decoder block. The output surface point cloud i.e., decoded features point cloud **405** is of size *Nₒᵤₜ* x *Fₒᵤₜ.*

A number of decoder blocks is a design choice and depends on the number of CSA blocks. Spatial dimension of output is also a design choice. Each decoder block requires input from the corresponding CSA block. Therefore, in the present embodiment the number of decoder blocks is seven.

It is common to gradually lower the number of output feature channels from previous blocks, usually using the next powers of two, or by dividing the number of channels by two. In the present embodiment seven decoder blocks are used as shown in the **Fig 7****.**

The first decoder block **716** gets an input from a previous decoder block **715,** and from the surface point cloud **702.** Its spatial output dimension is set to 64 x 256.

Decoder block **715** gets an input from a previous decoder block **714,** and from the CSA block **703.** Its spatial output dimension is set to 128 x 128.

Decoder block **714** gets an input from a previous decoder block **713,** and from the CSA block **704.** Its spatial output dimension is set to 256 x 128.

Decoder block **713** gets an input from a previous decoder block **712,** and from the CSA block **705.** Its spatial output dimension is set to 512 x 128.

Decoder block **712** gets an input from a previous decoder block **711,** and from the CSA block **702.** Its spatial output dimension is set to 1024 x 64.

Decoder block **711** gets an input from a previous decoder block **710,** and from the CSA block **707.** Its spatial output dimension is set to 2048 x 32.

The last decoder block **715** gets an input from CSA block 708 and CSA block **709.** Its spatial output dimension is set to *Nᵢₙ* x 128.

When the surface is fully decoded by the last decoder block, the point features are passed through the shared MLP blocks **206.**

The Shared MLP block is shown in **Fig. 5****.** The input to the Shared MLP block is an input surface point cloud **501** of spatial size *N* x *F.* The input surface point cloud is processed with 1D convolution **502** of specified number of output channels *C.* The output of the 1D convolution is then processed accordingly with 1D batch normalization layer **503,** activation function **504** and Dropout layer **505.** The output surface point cloud **506** is of spatial size *N* x *C.* The estimated hemodynamic parameter is determined by the parameter used in the training data set for training the network. If the ADNN is trained with FFR data - as in the present embodiment - then it estimates FFR data. On the other hand, the ADNN, according to embodiment of the invention, can be trained as well with other hemodynamic parameters, including pressure drops, wall shear stress and others.

The number of shared MLP blocks is a design choice.

In one embodiment only one shared MLP block **717** is used as shown in the **Fig 7****.** In one embodiment, the number of convolutional channels is set to 128.

The last step is to obtain the estimated per-point hemodynamic features output **207.** It includes applying a series of post-processing procedures. In one embodiment, a single 1D convolutional layer **718** is used to cast the output to a required number of points *Nᵢₙ* and outputs it in step **719.** Post processing may include, as in one embodiment casting output estimated hemodynamic parameters from points on the point cloud to points on a centerline graph or using these estimated hemodynamic parameters for further calculations in a CFD algorithm.

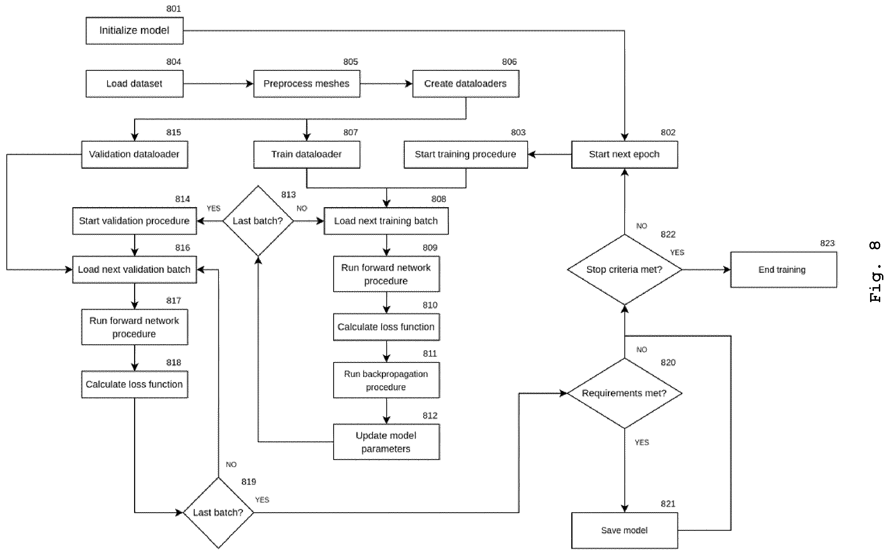

An embodiment of the method of training of an ADNN, according to the invention, is discussed below, in reference to **Fig. 8****.** The method comprises a step of obtaining of a set of training geometries in a form of surface mesh geometries or surface point cloud geometries.

Hemodynamic parameters used in this embodiment are pressure drops or FFR. A pressure drop or FFR corresponding to individual points in every training geometry is determined in CFD simulation to form a set of training ground-truth values.

Training data are formed by representing training geometries as surface point clouds and assigning pressure drops or FFR values to particular points in training geometries and then are used in training an ADNN model. Training data sets may comprise real data, artificial data, hybrid data or combination thereof and include patient-specific images, artery geometries and all related metadata.

The training procedure is adapted to the method of estimation. Generally, it comprises a step of obtaining of a set of geometries of vessel trees - either corresponding to real patients or to artificial models or both. Further, it includes obtaining values of parameter to be estimated by the trained network. The values may be obtained in real measurement or with CFD simulations or both. Subsequently, the training data for the ADNN are prepared using the geometries and the corresponding parameters including representing the geometries as a point cloud. This is crucial for the invention as point cloud representation with geodesic distance grouping allows avoiding the need of setting arbitrarily selected parameters. Then the ADNN is trained. The ADNN is an artificial deep neural network having an architecture adapted for point cloud processing with distance based point grouping. The distance is defined as geodesic distance along the blood vessel tree. Geodesic distance, preferably, is distance along the centerline, thus a step of obtaining a blood vessel tree centerline graph is required. Specific example of training procedure is described below in detail with reference to Fig. 8. It is noted though, that numerous alternative specific ways of training are available for the person skilled in the art, which are applicable as long as they meet requirements set out in claim 1.

The training procedure is preceded by model initialization **801** and preparing the input data **804.** The model initialization **801** includes initializing weights of the ADNN model which are sampled from a distribution of choice.

In one embodiment, in the load dataset step **804,** the samples - input surface meshes or point clouds and hemodynamic features to be regressed, are loaded into the memory. The samples are then preprocessed **805** - meshes are decomposed into centerline graphs and surface point clouds if needed. Then, additional features are computed and incorporated to each point on the point cloud as needed.

Once the data is preprocessed, the dataloaders are created **806** for both the train set **807** - on which the training is done, and for the validation set **815** - on which the model is evaluated during training procedure.

The model training comprises of a set of operations which are repeated iteratively - one such set is called an epoch. Each epoch **802** starts from the training procedure **803** which utilizes the train dataloder **807.** The dataloader yields batches of samples which are loaded into the memory **808** and processed with forward network procedure **809.**

When the network output is obtained, the loss function between the desired and yielded result is calculated **810.** In one embodiment, the loss function is the Mean Squared Error (MSE), and the loss is calculated for each point of the input surface point cloud and result is averaged. Once the loss is calculated, its gradient is used to perform backpropagation procedure **811,** which estimates how much the network weights need to be tweaked, to obtain a lower loss in the current epoch. According to the calculated loss gradients model parameters are updated **812.**

The set of instructions applied to the data batch is called a training step. The training steps are repeated until there are no data batches left in the train data loader **813.**

Once the last training batch has been processed, the validation procedure commences **814.** During the validation procedure, the model's weights are not changed. The validation process is performed to evaluate and monitor model's performance on a data that has not been included in the training set.

The validation step comprises of similar instructions as the training step - loading next data batch **816,** running forward procedure **817** and calculating loss function **818.** The validation step differs from the training step in the absence of backpropagation procedure and model's parameters update. The validation steps are done iteratively until all the validation batches have been yielded **819.**

Upon the validation procedure end, the requirements of the training process are checked **820.** Requirements may comprise of checking whether validation loss decreased compared to the previously lowest value. If it does, the ADNN model is saved **821.** Next, a stopping criterion **822** is checked. In one embodiment the stopping is defined as the number of epochs to be reached. If the stopping criterion is not met, the next epoch commences **802,** otherwise the training ends **823.**

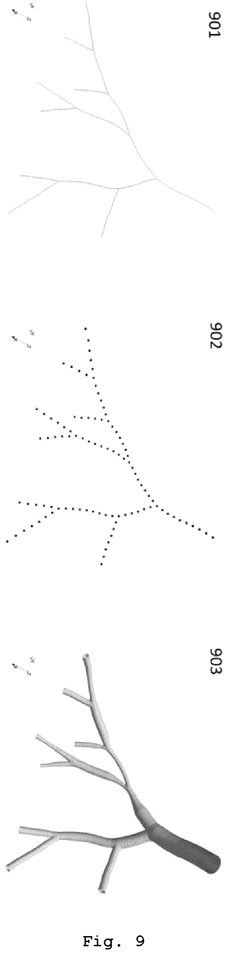

In the process of evaluating the invention, a dataset of 1,700 synthetically generated vessel geometries, in form of a surface mesh, was used. The training, validation and test set comprised of 1,500, 100 and 100 samples, respectively. In **Fig. 9****,** an example of a synthetic vessel is presented. A sample comprises of a centerline graph **901** (density of nodes showcased in **902**) and a mesh representing vessel geometry **903.**

An attempt to use distance based grouping with Euclidean distance instead of geodesic distance was unsuccessful and resulted in low correlation with results of CFD and real data.

The computers, in the sense of the above description, are to be understood as hardware devices including computers, microcontrollers, signal processing, programmable gate arrays, understood as hardware computers, graphic cards, application-specific integrated circuits, or other processing digital devices used for image processing, as well as distributed solutions including cloud computing environments.

Those skilled in the art, given the teachings of the above description, are able to routinely propose multiple hardware and software solutions for device, according to the invention, and for computer-implemented execution of the method, according to the invention, as well as methods of obtaining training information. Notably training and estimation can be done on the same computer systems or in completely separate computer systems. Use of trained system may include further training thereof.

It is noted that described invention is applicable for estimation of various hemodynamic parameters while FFR and pressure drops were given only by way of example. Estimated parameters depend on the parameters provided in the training data set.

It is stressed, that the above description is a mere illustration of the present invention and those skilled in the art will be able to propose many alternative embodiments covered by the scope of protection, as defined in the attached claims.

In the claims, any reference signs placed between parentheses shall not be construed as limiting the claim. The use of the verb "comprise" does not mean that there are no elements or steps other than those stated in a claim. The article "a" or "an" preceding an element does not exclude the presence of a plurality of such elements.

## Claims

1. A computer implemented method of training of an artificial deep neural network for estimation of a hemodynamic parameter from a geometry of a blood vessel tree of a patient, the method comprising
a step of obtaining a set of geometries of vessel trees,
a step of obtaining values of hemodynamic parameter corresponding to the geometries within the set,
a step of preparing the training data for the artificial deep neural network,
a step of training of the artificial deep neural network,
the step of preparing the training data for the artificial deep neural network comprises,
representing of the geometry as a point cloud,
**characterized in that**
the artificial deep neural network is an artificial deep neural network having an architecture adapted for point cloud processing with distance based point grouping,
wherein the distance is defined as geodesic distance along the blood vessel tree.

2. Computer implemented method according to claim 1, wherein obtaining values of hemodynamic parameters comprises using computational fluid dynamics simulation in the geometries within the set.

3. A computer implemented method of estimation of hemodynamic parameter from a geometry of a blood vessel tree for diagnosis of a patient using an artificial deep neural network, comprising a step of obtaining the geometry of the blood vessel tree **(103)** and a step **(105)** of applying the artificial deep neural network for estimation of the hemodynamic parameters, wherein the geometry is represented as a point cloud, **characterized in that** the artificial deep neural network is an artificial deep neural network having an architecture adapted for point cloud processing with distance based point grouping wherein the distance is defined as geodesic distance along the blood vessel tree

4. A computer program product comprising a set of instruction that when run on a computing system cause it to realize a computer implemented method according to any of claims 1-2.

5. A computer program product comprising a set of instruction that, when run on a computing system, cause it to realize a computer implemented method according to claim 3.

6. A computing system for extraction of at least one of estimation of hemodynamic parameters from a geometry of a blood vessel tree adapted to realize a computer implemented method as defined in claim 3.

7. A computing system according to claim 6, **characterized in that** it is further adapted to realize a computer implemented method of training, as defined in claim 1 or 2.

## Patentansprüche

1. Computerimplementiertes Verfahren zum Trainieren eines künstlichen tiefen neuronalen Netzwerks zur Schätzung eines hämodynamischen Parameters aus einer Geometrie eines Blutgefäßbaums eines Patienten, wobei das Verfahren umfasst
einen Schritt zum Erhalten einer Menge von Geometrien von Gefäßbäumen,
einen Schritt zum Erhalten von Werten des hämodynamischen Parameters, die den Geometrien innerhalb der Menge entsprechen,
einen Schritt zum Vorbereiten der Trainingsdaten für das künstliche tiefe neuronale Netzwerk,
einen Schritt zum Trainieren des künstlichen tiefen neuronalen Netzwerks,
**dadurch gekennzeichnet, dass**
das künstliche tiefe neuronale Netzwerk ein künstliches tiefes neuronales Netzwerk ist aufweisend eine Architektur, die für die Punktwolkenverarbeitung mit entfernungsbasierter Punktgruppierung angepasst ist,
wobei die Entfernung als geodätische Entfernung entlang des Blutgefäßbaums definiert ist.

2. Computerimplementiertes Verfahren nach Anspruch 1, wobei das Erhalten von Werten von hämodynamischen Parametern das Verwenden einer computergestützten Strömungssimulation in den Geometrien innerhalb des Satzes umfasst.

3. Computerimplementiertes Verfahren zur Schätzung eines hämodynamischen Parameters aus einer Geometrie eines Blutgefäßbaums zur Diagnose eines Patienten unter Verwendung eines künstlichen tiefen neuronalen Netzwerks, mit einem Schritt zum Erhalten der Geometrie des Blutgefäßbaums **(103)** und einem Schritt **(105)** zum Anwenden des künstlichen tiefen neuronalen Netzwerks zur Schätzung der hämodynamischen Parameter, wobei die die Geometrie als Punktwolke dargestellt wird,
**dadurch gekennzeichnet, dass**
das künstliche tiefe neuronale Netzwerk ein künstliches tiefes neuronales Netzwerk ist aufweisend eine Architektur, die für die Punktwolkenverarbeitung mit distanzbasierter Punktgruppierung angepasst ist,
wobei die Distanz als geodätische Distanz entlang des Blutgefäßbaums definiert ist.

4. Computerprogrammprodukt, das einen Satz von Anweisungen umfasst, die, wenn sie auf einem Computersystem ausgeführt werden, dieses veranlassen, ein computerimplementiertes Verfahren gemäß einem der Ansprüche 1 bis 2 zu realisieren.

5. Computerprogrammprodukt, das einen Satz von Anweisungen umfasst, die, wenn sie auf einem Computersystem ausgeführt werden, dieses veranlassen, ein computerimplementiertes Verfahren gemäß Anspruch 3 zu realisieren.

6. Computersystem zur Extraktion mindestens einer Schätzung hämodynamischer Parameter aus einer Geometrie eines Blutgefäßbaums, das zur Realisierung eines computerimplementierten Verfahrens gemäß Anspruch 3 geeignet ist.

7. Computersystem gemäß Anspruch 6, **dadurch gekennzeichnet, dass** es ferner dazu ausgelegt ist, ein computerimplementiertes Trainingsverfahren gemäß Anspruch 1 oder 2 zu realisieren.

## Revendications

1. Procédé mis en œuvre par ordinateur pour l'entraînement d'un réseau neuronal profond artificiel pour l'estimation d'un paramètre hémodynamique à partir de la géométrie d'une arborescence de vaisseaux sanguins d'un patient, le procédé comprenant
une étape d'obtention d'un ensemble de géométries d'arborescences de vaisseaux,
une étape d'obtention des valeurs du paramètre hémodynamique correspondant aux géométries à l'intérieur de l'ensemble,
une étape de préparation des données d'entraînement pour le réseau de neurones profonds artificiels,
une étape d'entraînement du réseau de neurones profonds artificiels,
**caractérisé en ce que**
Le réseau de neurones profonds artificiels est un réseau de neurones profonds artificiels ayant une architecture adaptée au traitement de nuages de points avec regroupement de points basé sur la distance,
où la distance est définie comme la distance géodésique le long de l'arborescence des vaisseaux sanguins.

2. Procédé mis en œuvre par ordinateur selon la revendication 1, dans lequel l'obtention de valeurs de paramètres hémodynamiques comprend l'utilisation de la simulation numérique de la dynamique des fluides dans les géométries de l'ensemble.

3. Procédé mis en œuvre par ordinateur d'estimation d'un paramètre hémodynamique à partir de la géométrie d'une arborescence de vaisseaux sanguins pour le diagnostic d'un patient à l'aide d'un réseau de neurones profonds artificiels, comprenant une étape d'obtention d'une géométrie d'une arborescence de vaisseaux sanguins **(103)** et une étape **(105)** d'application d'un réseau de neurones profonds artificiels pour l'estimation de paramètres hémodynamiques, dans lequel
la géométrie est représentée par un nuage de points, **caractérisé en ce que**
Le réseau de neurones profonds artificiels est un réseau de neurones profonds artificiels ayant une architecture adaptée au traitement des nuages de points avec regroupement de points basé sur la distance
dans lequel la distance est définie comme la distance géodésique le long de l'arborescence des vaisseaux sanguins.

4. Produit programme d'ordinateur comprenant un ensemble d'instructions qui, lorsqu'elles sont exécutées sur un système informatique, lui permettent d'exécuter un procédé mis en œuvre par ordinateur conformément à l'une quelconque des revendications 1 à 2.

5. Produit programme d'ordinateur comprenant un ensemble d'instructions qui, lorsqu'elles sont exécutées sur un système informatique, lui permettent d'exécuter un procédé mis en œuvre par ordinateur conformément à la revendication 3.

6. Système informatique pour l'extraction d'au moins une estimation de paramètres hémodynamiques à partir de la géométrie d'une arborescence de vaisseaux sanguins adapté pour exécuter un procédé mis en œuvre par ordinateur, tel que défini dans la revendication 3.

7. Système informatique selon la revendication 6, **caractérisé en ce qu'**il est en outre adapté pour exécuter un procédé d'entraînement mis en œuvre par ordinateur, tel que défini dans la revendication 1 ou 2.
